(19) Europäisches Patentamt
European Patent Office
Office européen des brevets

(11) **EP 2 229 907 A1**

(12) **EUROPEAN PATENT APPLICATION**

(43) Date of publication:
**22.09.2010 Bulletin 2010/38**

(51) Int Cl.:
***A61B 18/18*** (2006.01)

(21) Application number: **10166292.2**

(22) Date of filing: **10.09.2007**

| | |
|---|---|
| (84) Designated Contracting States:<br>**AT BE BG CH CY CZ DE DK EE ES FI FR GB GR HU IE IS IT LI LT LU LV MC MT NL PL PT RO SE SI SK TR** | (72) Inventors:<br>• **Lukowiak, Marc**<br>  **21000, Dijon (FR)**<br>• **Lavigne, Christophe**<br>  **F-01170, Gex (FR)** |
| (30) Priority: **08.09.2006 US 842943 P**<br>        **01.08.2007 US 882453** | (74) Representative: **Lecca, Patricia S.**<br>**Cabinet Lecca**<br>**21 rue de Fécamp**<br>**75012 Paris (FR)** |
| (62) Document number(s) of the earlier application(s) in accordance with Art. 76 EPC:<br>**07820101.9 / 2 066 252** | |
| (71) Applicant: **Renewave Medical Systems SA**<br>**1260 Nyon (CH)** | Remarks:<br>This application was filed on 17-06-2010 as a divisional application to the application mentioned under INID code 62. |

(54) **Microwave devices for treating uterine tissue**

(57)    The invention relates to a uterine tissue treatment device or energy delivery device and the associated tip device for contacting skin or tissue. The devices can be advantageously used to treat endometriosis. A number of systems or control systems can be incorporated into the devices to afford manual or automatic control of the energy delivered. In preferred embodiments, microwave range energy is used in the treatments.

FIG. 1

EP 2 229 907 A1

## Description

This application claims the benefit of United States application 11/882,453, filed August 01, 2007, and United States provisional application no. 60/842,943, filed September 08, 2006, which are hereby incorporated by reference as though fully set forth herein.

FIELD OF INVENTION

The invention relates to electronic devices and methods for using them to non-invasively treat or affect tissues. In particular, the devices can be used to deliver an effective amount of energy, typically microwave energy, from the surface of skin, such as the face, to the underlying layers of tissue in order to reduce wrinkles, fine lines, fissures, and/or skin discoloration or marks. In other aspects, the invention relates to devices and methods for effectively concentrating the effects of microwave energy to treat a selected volume of tissue.

BACKGROUND OF INVENTION

While the use of microwave energy to treat skin has been proposed in the past, methods to effectively reduce facial wrinkles and other age-related blemishes have not been in use. Generally, microwave devices have been implemented in catheter-based and invasive methods, which are distinctly disadvantageous compared to non-invasive methods. Accordingly, improved microwave devices and methods for noninvasive treatment of tissue are desired in the art.

BRIEF SUMMARY OF THE INVENTION

The invention provides an energy delivery device for treating tissues, particularly skin tissues and wrinkled, contoured, or fissured skin. The device comprises a hand held energy delivery device (EDD) with a removable tip that directs energy, originating from a sinusoidal wave generator (in the microwave frequency range), amplifier and waveguide, controlled by a computer, into the wrinkle, fissure, etc. The energy is given in pulses, the duration of which can be varied, for example, in the range of 1ms to 1 sec, according to the type of treatment, type of skin to be treated, etc. The pulse interval can also be controlled in a similar range. The power of the pulsed waves, supplied by the generator through the amplifier, is in the range of 0.1 - 100 Watts. The applied wave parameters can also be controlled by feedback loop activated by sensors monitoring the skin condition. These sensors can be for temperature (thermocouples, thermistors, radiometers), reflected wave measuring system, optic, etc. Optionally, a cover around the EDD tip can be used to flatten the skin surface when in contact with it, or within which a negative (vacuum) pressure can be created to flatten skin tissue for the tip to more directly apply energy to the skin and the layers of tissue below the skin surface.

In one aspect, the devices and methods of the invention address the problem of existing skin treatments, where deleterious or uncomfortable side effects or burning are present, by providing a more focused, easier to control, and less harmful alternative. In another aspect, the devices and methods of the invention address the problem of directing skin or tissue treatment to specific areas, such as within wrinkles, fissures or other blemishes. While skin treatments are a preferred aspect, the invention is not limited to uses on external surfaces or skin.

Thus, the invention comprises in one embodiment a device for treating wrinkles, fines lines, or blemishes on skin, where the device comprises a handheld housing unit with a treatment tip and a passage for a waveguide connecting to a connection point, where the waveguide exits the housing at a grip end and energy can be delivered to skin via a microwave emitting treatment tip. The distal end of the treatment tip is capable of contacting the surfaces of a wrinkle or fine line or blemish in the skin, and the treatment tip is functionally connected to the connection point to transfer energy from the waveguide to the distal end of the tip contacting skin. The device further comprises a control system for varying the pulse length, frequency, and amplitude of the energy delivered. In a preferred embodiment, the energy is in a microwave frequency range of about 300 MHz to about 30 GHz, and/or the energy pulses are designed to create a heating effect in the tissue below the surface of the skin. The effect of the treatment, when healed after the heating, is a reduction in the appearance of wrinkles, fine lines, or blemishes and there is little to no damage to the surface of the skin. Several preferred options are disclosed in detail and can be incorporated into any device or methods of the invention, such as: a microwave emitting treatment tip that is replaceable or that comprises a replaceable cover; the use of the device where bipolar microwave power is used; the incorporation of a control system that measures the energy reflection during treatment of the skin in order to adjust one or more of the amplitude, pulse number, pulse duration, or frequency of the energy delivered to the treatment tip; a connection point that contains a dielectric composition; a treatment tip that contains a dielectric composition; and the use of a dielectric composition that comprises Teflon in the treatment tip and/or connection point; a treatment tip with a convex distal tip to contact skin; a treatment tip with a pointed distal tip to contact skin; an electronic delivery device tip with a tapered point at a distal end; and an electronic delivery device tip surrounded by a cover, wherein a negative pressure can be applied within the cover when the electronic delivery device tip is in contact with the skin tissue.

The device can be operated, or the control system can be design to operate, with a variety of programmed or set features or functions. For example, the impedance of a dielectric composition used can be selected to deliver energy to skin in the frequency range of about 433 MHz to about 5800 MHz. Microwave pulses can be selected, for example of about 1 msec or more in length and of a desired frequency and energy to penetrate to a desired depth into tissue. The control system and method can detect reflected waves and control the frequency, energy or both to the energy delivery device tip. Furthermore, the device can comprise one or more shielding regions that surround a substantial part of the treatment tip. In one embodiment, the distal end of the treatment tip extends between about 0.1mm and about 5 mm beyond a shielding region, and in another the distal end of the treatment tip extends about 1 mm beyond a shielding region. The device or method can employ a treatment tip configured to, or operated under conditions to, generate a microwave field within the tissue having a volume of about 10 mm$^3$.

Any of the methods or devices of the invention can be used at or contain controls to be used to emit microwaves that applied to skin tissue are less than about 10 Watts. Alternatively, or in addition, the methods and devices can employ a frequency of emitted microwaves at about 2.45 GHz.

As noted, the device or method can include a negative pressure device, so that the electronic delivery device tip is engaged within a wrinkle, contour, or fissure of the skin tissue while the negative pressure is applied. In any aspect of the device or methods of the invention, the electronic delivery device tip can be selected to deliver microwaves to effect the heating of tissue at a point or area about 0.5 mm to about 2 mm below the external surface of skin. Similarly, the electronic delivery device tip can be selected to deliver microwaves to affect the heating of a volume of tissue of about 10 mm$^3$.

In any embodiment of the invention, at least one secondary tissue treatment device can be incorporated or used. Several secondary tissue treatment devices are known in the art and preferred ones are selected from the group consisting of laser tissue treatment devices, IPL tissue treatment devices, and radiofrequency tissue treatment devices. Fore example, a variety of lasers and wavelengths or ranges or wavelengths have been used in the past to effect or alter skin conditions, including between 300 nm and 2000 nm. Thus, the invention is not limited to the specific examples and description of specific or preferred uses or elements described here, and one of skill in the art can device numerous permutations without departing from the invention as a whole.

BRIEF DESCRIPTION OF THE DRAWINGS

The patent or application file contains at least one drawing executed in color. Copies of this patent or patent application publication with color drawing(s) will be provided by the Office upon request and payment of the necessary fee.

For a more complete understanding of the invention and some advantages thereof, reference is now made to the following descriptions taken in connection with the accompanying drawings in which:

Figure 1 schematically represents the EDD.
Figure 2 represents an exploded view of the EDD in order to show its components.
Figure 3 represents a cross-sectional view of the EDD and describes the possible connections between the energy conduit or waveguide through the replaceable unit or tip, or applicator tip or antenna body, and the coaxial cable.
Figure 4 represents means for interlocking the core (14) of the coaxial cable and the core (5) of the replaceable unit.
Figure 5 illustrates relative dimensions of the core (5) of the replaceable unit and the dielectric (6).
Figure 6 represents a screw-on or bolt assembly or mechanism between the handheld portion and the tip portion of an EDD.
Figure 7 represents a bayonet connection between the handheld portion and the tip portion of an EDD.
Figure 8 represents a clamping mechanism between the handheld portion and the tip portion of an EDD.
Figure 9 represents an assembly whereby a click mechanism connects the handheld portion and the tip portion of the EDD.
Figures 10 through 12 illustrate steps that may be carried out in removing the tip portion from the handheld portion.
Figure 13 depicts an exemplary block diagram of the system of the invention and its operation.
Figure 14 shows an example of a software simulated energy density field of the energy delivery device in tissue.

DETAILED DESCRIPTION OF THE INVENTION

In one aspect, the invention comprises a microwave emitting or delivery device, referred to herein as an energy delivery device (EDD), that provides a controlled delivery of energy to the skin and/or its underlying layers. The use of the device of the invention induces regeneration of the skin and particularly areas having collagen fibers. Typically, the degree of fibrous bundling or cross-linking in these areas increases with age, resulting in fine lines, wrinkles, and eventually pronounced fissures or furrows. In general, the devices and methods of the invention and the energy delivery device are used in direct contact with the surface of the skin and the emitted energy affects tissue below the surface of the skin, especially the underlying collagen fiber layer, such as the dermis or epidermis. Treatment of the collagen fibers affects the cross-links in the fibrous material causing it to heal or regenerate. The healing or regenerating tissue will impart an improved appearance to the surface of the skin, removing wrinkles and fine lines. Thus, the appearance of desired areas or portions of the skin's surface are improved.

In a preferred embodiment, the method of the invention encompasses a treatment mode wherein the tissue is skin and the emitted microwaves are directed to an area of the body where aged or wrinkled skin is present, such as around the eyes, lips, chin, neck, and forehead.

The devices and methods of the invention can be adapted and used with a variety of tissues and other targets at a variety of energy penetrating depths. While skin tissue is discussed in general here, other tissue can also be treated. For example, the devices and methods disclosed herein may be utilized to good advantage on uterine tissue and/or in the treatment of endometriosis, incontinence, urinary incontinence, and disorders related to these, so that, for example, laparoscopic, minimally-invasive, intra-vaginal and/or and intra-uterine delivery devices or tips and methods of using them are specifically encompassed by the invention. Thus, while the use on skin tissue is discussed in particular, the invention is not limited to use with any particular tissue or target.

In preferred aspects, the invention comprises an EDD tip or probe. Once a target area has been identified for treatment, the energy delivered depends on the patient, the type of skin, the size of the wrinkles or other skin blemish or area, and the desired or appropriate temperature for the treatment. The duration of the pulses of the microwave energy of the preferred wavelength is chosen for the type of treatment desired. Depending on the treatment desired, a microwave pulse can be about 1 msec in length, or between about 1 msec and 100 msec, or between about 1 msec and 10 msec.

In a generic version of the microwave emitting device, the power supplied from the microwave generator or source can be applied in a range of about 0.1-100 Watts per probe or tip and, preferably, in a range of about 1-15 Watts per tip. While preferably only one tip is used, more than one can also be used. A coaxial cable of 50 Ohm can be used to supply power to the EDD tip or tips. The power may be applied in short high power pulses, preferably in the Microwave frequency range of about 300MHz-30 GHz, and most preferably at a wave frequency of about 2.45 GHz. Treatment is continued for a desired length of time in accordance with the desired results.

The amount of energy delivered to disrupt or break the fibrous bonds depends on the condition of the skin. An electromagnetic field, particularly in the microwave region between 300MHz and 10 GHz, is effective at treating these fibrous layers. In various embodiments, the microwave delivery device and treatment regimen provides a controlled delivery of electromagnetic energy to the skin. The control system employed can comprise or include a pulse controller for selecting the desired average power and duration of the energy pulse delivered to the EDD tip. The control system employed is used for selection of the frequency, pulse width and amplitude, pulse interval, etc. The microwaves are generated by different oscillators delivering a sinusoidal signals at a frequency in the range of about 433 MHz to about 5800 MHz to the EDD. The pulse duration can be controlled, but preferably about a 10 msec pulse, the intensity of which is in the range of about 0.1-20 W, is used. A standing wave ratio indicator can be used to both adjust the position of the EDD or the pressure the EDD exerts on the skin, and to control the power transmitted to the skin so as to adjust the required transmitted power to the skin.

In optimal procedures, the skin, at the treatment area, is gently straightened or flattened, for example by hand or by one or more appropriate devices that do not interfere with the microwave delivery. The energy is thus delivered into, or as close as possible to, the wrinkled area and/or inside the flattened area of a wrinkle or fissure. In other optional embodiments, a vacuum can be used around the EDD tip so that the skin is flattened by the vacuum action

In one example of the devices and methods of treating skin or layers of skin, the EDD tip receives microwave energy from a source fed through a flexible, coaxial line. A single tip is generally used, where the tip is either pointed or blunt-ended at the distal end designated to be in contact with the skin during use. In a preferred embodiment, the tip is a shielded, directional emitter of microwave energy having a central microwave conductor, such as a rigid wire, that terminates in a blunt shape at the distal end, and where the distal end extends beyond a metallic shield that surrounds a portion of the conductor or rigid wire. One or more dielectric compounds or media with appropriate dielectric properties, such as Teflon, is positioned in the space between the shield and the rigid wire. The shield design and shape can vary and the distance between the distal end of the central conductor and the shield can vary, but a preferred distance is between about 0.1 mm and 5 mm.

The proximal end of the conducting tip is functionally connected to the waveguide originating in the generator (e.g., via a coaxial cable). The features of the handheld unit can include an ON/OFF switch and an intensity control knob or actuator for controlling the pulse, for example the pulse length and/or pulse energy. The length of the tip is generally designed for use at one or more frequencies, and as noted a preferred frequency is about 2.45 GHz. Various frequencies and tip lengths can be selected and a system incorporated in the handheld unit can be structured to accommodate different, interchangeable tips that connect to the same waveguide and controllable generator.

In a preferred embodiment, the tip is disposable and can be removed and replaced for use with different patients.

A connection point within the handheld unit, for example, connects the coaxial cable or other flexible waveguide to the rigid waveguide ending in the tip. Also, the tip can have differing diameters in the range of about 0.5mm to 5mm. More generally and as noted, the distal end of the tip can comprise a short tapered shape, a conical shape, or any other size and/or shape that can be inserted into a wrinkled region or fissure in the skin to be treated. The tip can also include a dielectric compound to provide impedance matching with the treatment area. Optionally, a tube carrying gas or liquid can extend from the handheld unit, along the waveguide and within the unit housing, to provide cooling gas or liquid to the treatment site. A similar tube can be used for suction to provide a negative pressure at the treatment site. The gas, fluid, and/or vacuum lines can be controlled, according to the amount of energy delivered, or controlled independently.

An exemplary system for the handheld microwave energy delivery device comprises a handheld unit coupled to one or more flexible lines that are functionally connected to a generator or source of pulsed microwave energy, gas, or liquid supply, vacuum, and a computer-controlled circuit to control the timing, frequency, power and pulse duration of the microwave energy. The computer-controlled circuit can also be used to processes or monitor and optimize operation using feedback data such as the reflected standing wave ratio, as known in the art. The treatment of different skin conditions and areas can involve differences in reflected waves, and adjusting one or more of several parameters can control the energy delivery from the tip of the device. The handheld unit can consist of a housing and switches for initiating and terminating the pulse, adjusting the frequency, power or pulse duration, for initiating and terminating vacuum, and optionally for cooling air or liquid supply. Indicator lights on the handheld housing can also be connected to the reflected energy monitor, or other measuring sensor in the system.

Preferably, the EDD device employs bipolar microwave energy delivery to affect a small treatment area and/or ensure consistent delivery of energy to the skin. However, unipolar and combination of unipolar/bipolar devices can also be used according to the invention. A temperature sensor (e.g., a thermocouple of thermistor) can optionally be incorporated at the distal part of the EDD device, and the sensor can be linked to a control device so as to control or limit the heating at the skin surface.

Other embodiments and advantages of the invention are set forth in part in the description that follows, and in part, will be understood from this description, or may be learned from the practice of the invention.

For a more complete understanding of the invention and some advantages thereof, reference is now made to the following descriptions taken in connection with the accompanying drawings.

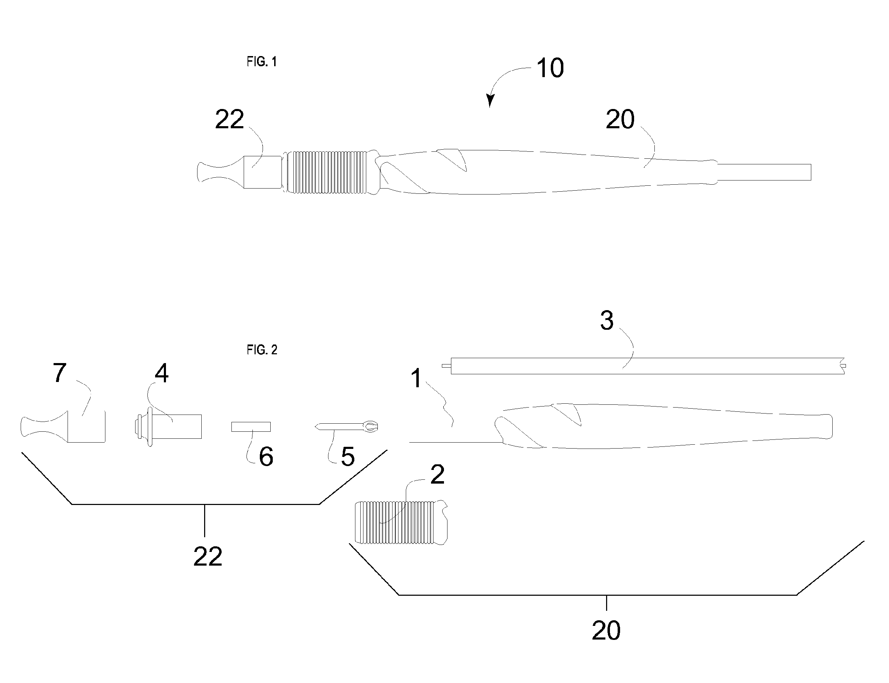

Figure 1 schematically represents the EDD 10 as a handheld unit including a handheld housing 20 and a tip portion 22. Figure 2 represents an exploded view of the EDD 10 in order to show the components.

Handheld housing 20 includes the main body (1), which can be of plastic or other materials, and which is designed to have an ergonomic form to hold by hand. The sleeve (2) is generally textured and formed to fit comfortably in the hand without sliding. The coaxial cable (3) connects the EDD 10 (in particular, the metallic core 5) to the base station (not shown).

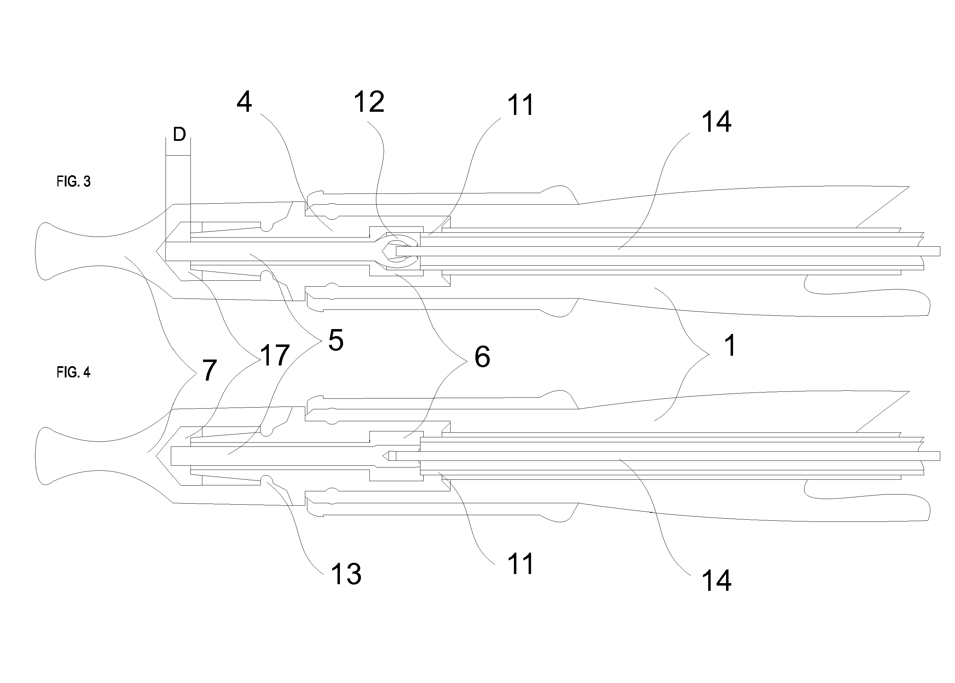

The replaceable EDD tip portion 22 unit is generally composed of a metallic antenna body (4) (preferably silver coated copper), a metallic core (5) (preferably silver coated copper), and a dielectric (6) (preferably Teflon) disposed between the metallic core 5 and the antenna body 4 (seen in Figures 3 and 4). A protective cap (7) can be used to protect the tip portion 22 of the EDD 10.

Figures 3 and 4 are cross-sectional views of the EDD 10 illustrating possible connections between the replaceable tip unit 22 of the EDD 10 and the coaxial cable (3). The coaxial cable (3) is held in the main body (1). The shielding (11) of the coaxial cable (3) is in contact with the antenna body (4). Figure 3 represents a gripping or snap-on mechanism (12) between the core (14) of the coaxial cable (3) and the core (5) of the replaceable unit. Figure 4 represents a connection by interlocking the core (14) of the coaxial cable and the core (5) of the replaceable unit. Of course, other methods of connecting the coaxial cable 3 and the core 5 are within the spirit and scope of the present invention.

The core (5) of the replaceable EDD tip 22 can be of different diameters (e.g., about 0.5mm to 5mm) depending on the tissue to be treated. The protective cap (7) can be fixed to the tip or antenna body by a click-on design or mechanism (13), a threaded connection, a friction fit, or any other suitable method. The protective cap (7) protects the tip of the core (5) from mechanical damage or contamination. The protective cap (7) can be made out of an insulating material.

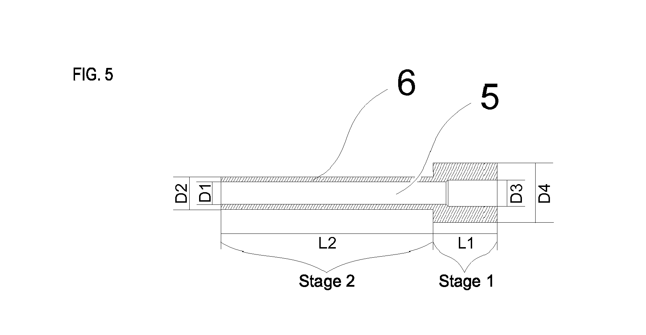

Figure 5 illustrates the relative dimensions of the core 5 and the dielectric 6. In use, the metallic core 5 of the EDD tip 22 is in contact at its distal end with a complex impedance $Z_b$, which is the impedance of the contact plan between the EDD tip 22 and the skin being treated. $Z_b$ is highly frequency dependent. At its proximal end, the core 5 is in contact with the coaxial cable 3, for which the impedance may be represented as $Z_0$. $Z_0$ is typically about 50 Ohms. One function of metallic core 5 is impedance matching-that is, to enable efficient transmission of the wave from $Z_0$ to $Z_b$. For this matching, the core 5 may be constructed out of two stages, as shown in Fig. 5. Stage 1 is of length $L_1$ and has a characteristic impedance $Z_1$. Stage 2 is of length $L_2$ and has a characteristic impedance $Z_2$.

As known in the art, the impedance of a coaxial wave guide depends on the permittivity and the inner and outer diameters of the dielectric. This impedance is frequency independent, and can be calculated using the equations

$$Z_1 = \frac{60}{\sqrt{\varepsilon_r}} \ln\left(\frac{D_4}{D_3}\right) \quad \text{and} \quad Z_2 = \frac{60}{\sqrt{\varepsilon_r}} \ln\left(\frac{D_2}{D_1}\right)$$, where $D_3$ and $D_4$ are, respectively, the inner and outer diameter of the Stage 1 of the dielectric and $D_1$ and $D_2$ are, respectively, the inner and outer diameter of Stage 2 of the dielectric.

The first step in calculating the dimensions of the two stages of core 5 is to determine the impedance $Z_b$. For this, a coaxial cable with a chosen length of L, chosen diameters $D_1$ and $D_2$, and of characteristic impedance $Z_2$ (typically about 50 Ohms) may be attached to a network analyzer. As shown in Figures 3 and 4, the core 5 is longer than the antenna body 4 by a distance D, which is preferably about 1mm. This "tip" (e.g., the exposed length of core 5) may be placed on the skin, and, using the network analyzer to measure impedance, $Z_b$ may be calculated according to the equation $Z_b = Z_{measured}e^{2\gamma L}$ where $\gamma = j * \frac{2\pi}{\lambda}$, where $\lambda$ is the wavelength in the dielectric having permittivity $\varepsilon_r$.

The second step is to calculate the length $L_2$ of Stage 2. $L_2$ is preferably calculated in order to define a characteristic impedance $Z_c$ of the interface between Stage 1 and Stage 2 that is real only (e.g., lacking an imaginary component). $L_2$ can be found using the equation $Z_c = Z_b e^{-2\gamma L2}$.

The third and final step is to match $Z_c$ with $Z_0$ (e.g., about 50 Ohms). Since $Z_c$ is designed to be real-only, an impedance transformer can be used. The length $L_1$ of Stage 1 is preferably a quarter wavelength (e.g., $\lambda/4$), and $Z_1$ is given by the equation $Z_1 = \sqrt{Z_c * Z_0}$.

In order to perform the above calculations, an electromagnetic field distribution simulation software may be used. In particular, it is desirable for the electromagnetic field distribution simulation software to account for each discontinuity between stages. The length of core 5 can be reduced by using a dielectric with intermediate or high permittivity. For example, in order to calculate $L_2$, one needs to know $Z_b$. In order to have a real-only $Z_c$, two solutions of $L_2$ are possible. Preferably, the solution which gives the minimum value of $Z_c$ is chosen. The following equation may be employed: $Z_1 = \sqrt{Z_{c_{min}} * Z_0}$, where $Z_{c_{min}} = Z_0 * \frac{1 - |\Gamma|}{1 + |\Gamma|}$, and where $\Gamma$ is the reflection coefficient of the core 5 as measured by the network analyzer. The following table provides some representative values for the core 5.

| Frequency | $\varepsilon_r$ | $L_1$ (mm) | $L_2$ (mm) | $Z_1$ (Ohms) | $D_4/D_3$ |
|---|---|---|---|---|---|
| 433 MHz | 9 | 57 | 114 (max) | 13 | 1.9 |
| 868 MHz | 9 | 29 | 58 (max) | 17 | 2.3 |
| 2.45 GHz | 9 | 10 | 20 (max) | 21 | 2.8 |
| 5.8 GHz | 9 | 4 | 8 (max) | 30 | 4.5 |

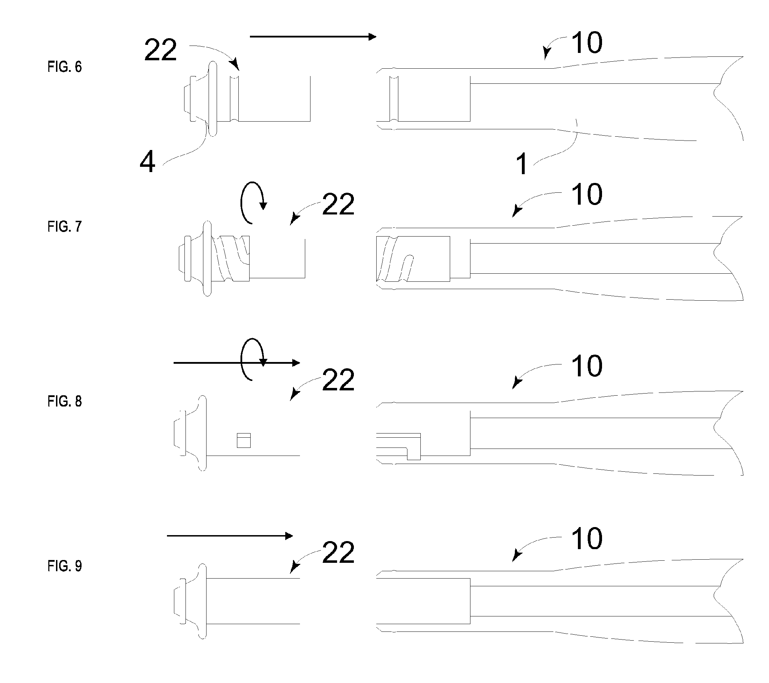

Figures 6-9 represent possible assemblies between the handheld portion 20 and the tip portion 22. Figure 6 represents an assembly comprising a snap or click mechanism between the tip portion 22 and the handheld portion 20. Figure 7 represents a bolt assembly or mechanism between the tip portion 22 and the handheld portion 20 Figure 8 represents a bayonet-type connection between the tip portion 22 and the handheld portion 20. Figure 9 represents a clamping-type connection between the tip portion 22 and the handheld portion 20.

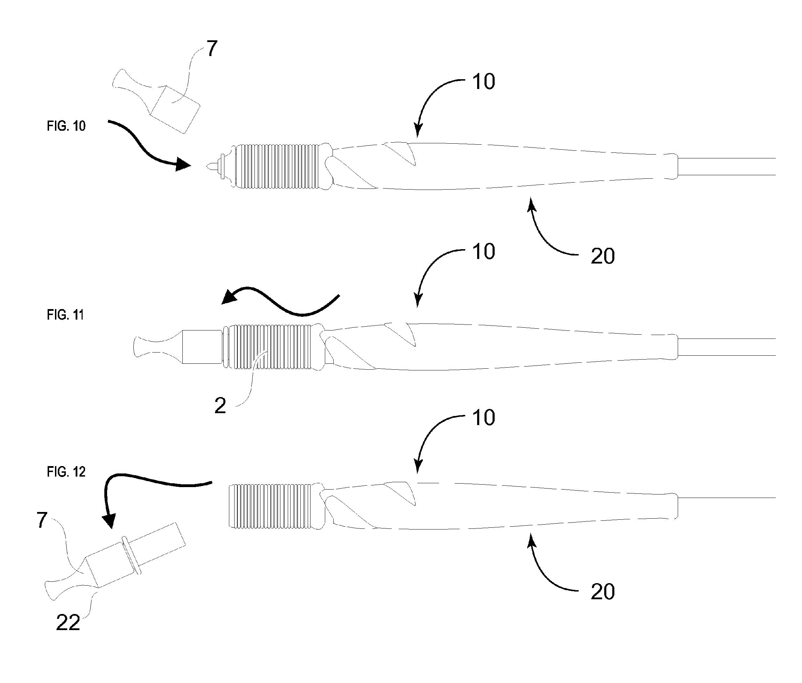

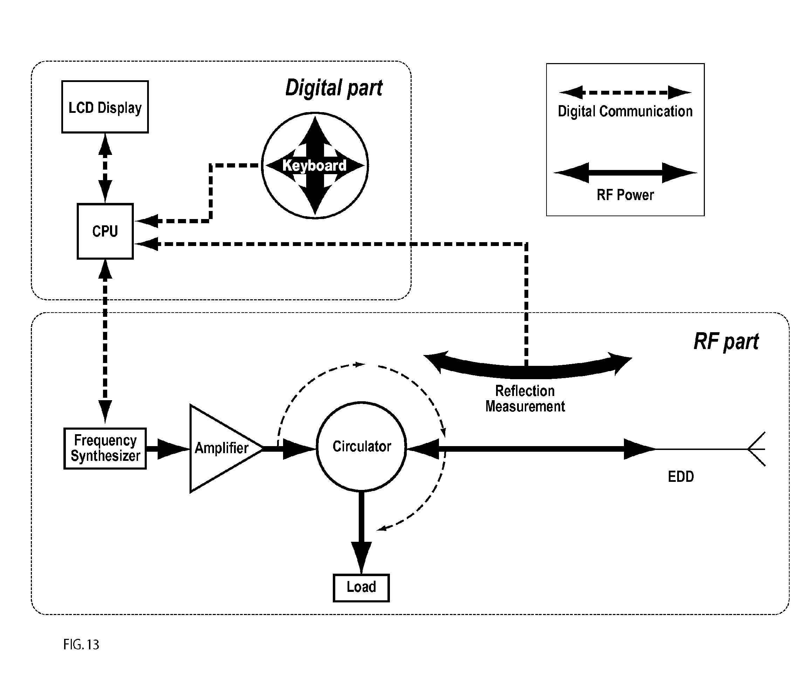

Figures 10-12 illustrate steps that may be carried out in detaching the tip portion 22 from the handheld portion 20. Figure 10 represents an assembly whereby a snap or click mechanism connects the protective cap (7) to the EDD 10. Figures 11 and 12 represent the removal/extraction of the tip portion 22. By following the illustrated steps, a user can extract the tip portion 22, including core 5, antenna 4, and dielectric 6, without touching it. Figure 13 is a block diagram of an exemplary complete system of the invention and its operation. The distal end RF part relates to the EDD tip, here depicted by the symbol for an antenna.

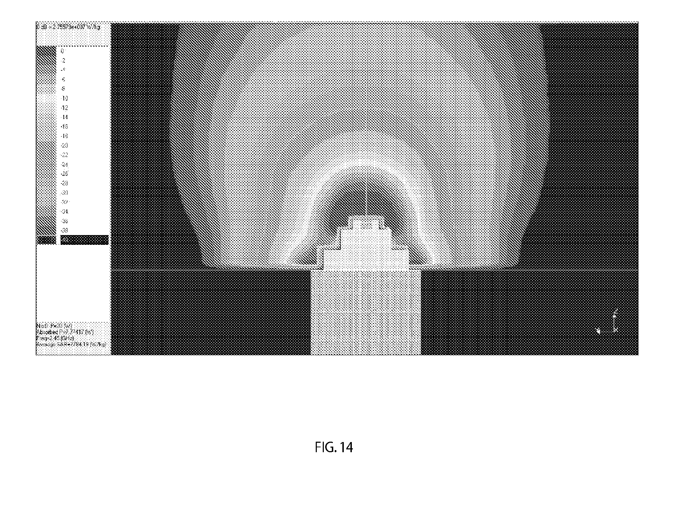

Figure 14 illustrates an exemplary energy density field of the energy delivered by EDD 10 in tissue. The power of the microwaves delivered to the skin can be controlled manually or automatically. The automatic control may be based on an optional feedback loop that is activated, for example, by one or more of the following sensory devices: skin temperature measurement or monitoring by thermocouples, thermistors, or IR optic sensors, reflected wave monitoring system, and the like. The temperature of the tissues underlying the skin surface can be monitored by radiometers or IR optic sensors, for example, or other mechanisms or methods known or available in the art. The feedback system preferably adjusts the microwave power in order to obtain optimal treatment of the specific tissues without overheating that may cause damage. A timer can also be set to prevent excess treatment. It is known in the art that the depth of penetration of microwaves into tissue is frequency dependent. The table below gives the depth of penetration as a function of frequency for tissues with high water content (e.g., muscle, internal organs such as the liver and the heart, and connective tissue with little fat).

| Frequency MHz | Conductivity S/cm | Penetration depth Mm |
| --- | --- | --- |
| **10** | 0.625 | 200 |
| **100** | 0.889 | 53 |
| **300** | 1.37 | 25 |
| **915** | 1.6 | 13 |
| **2450** | 2.21 | 6.8 |
| **10000** | 10.3 | 1.6 |

In view of the above, the frequency of the microwaves can be varied so as to more effectively treat the tissues at the desired depth. This goal can be achieved by waves that carry a number of frequency components or by alternatively mixing pulses of different frequency.

As described, the EDD tip portion 22 can be replaced. This property provides distinct functions or advantages, for example enabling the user to select and use the tip portion optimal to the specific need and/or to replace the tip portion for each new patient. The size and/or shape of the terminal (e.g., distal) part of the EDD tip portion may vary according to need: it can be flat, curved, conical, or have the form of a cylinder running parallel to the skin surface. Such a cylinder tip can, for example, be placed along a wrinkle to obtain optimal treatment of an elongated or long target area with one "shot" or application. Preferably, the tip portion is designed in order to generate a microwave field that is generally restricted to a volume of about 10 mm$^3$, taking into consideration both the size and shape of the tip portion as well as the tissue penetration of the selected microwave frequency.

To avoid the contamination of the skin of one patient by the use of the same tip on more than one patient, the tip can be covered by a specially constructed protection cover that does not interfere with the spread of the microwave field from the tip to the tissues. The protection cover can be made of a dielectric having dielectric properties that ensure impedance matching between the tip and the tissues. Alternatively the protective cover can have electric conductivity similar to that of the skin but be impermeable to bacteria and viruses as well as other contaminants. This could be, for example, a hard gel, wet cellophane, or the like. A material that improves the tip - tissue impedance matching, such as a gel with the proper dielectric properties, can optionally be added to the gap between the tip and the underlying skin. Of course, as described above, it is also contemplated that the tip may be replaced between patients.

The following Examples and forgoing description are intended to show merely optional configurations for the devices of the invention. Variations, modifications, and additional attachments can be made by one of skill in the art. Thus, the scope of the invention is not limited to any specific Example or any specific embodiment described herein. Furthermore, the claims are not limited to any particular embodiment shown or described here.

**Examples - Dermatology Treatment**

In a first example, a shielded, directional tip is used on a device of the invention and applied directly against the surface of the skin without water, hydrating solutions, or other liquids. The tip has a blunt end to maximize the contact surface of the conductor with the skin. The aperture or distance between the shield and the tip (e.g., dimension D in Figure 3) can be varied by changing tips, thereby changing the energy or field shape or size. A preferred aperture is about 1mm. The tip is preferably a silver coated copper wire, but can be coated, for example with gold to prevent skin reactions in sensitive skin. In another example of a device in accordance with the invention, the distal end optionally includes a vacuum (negative pressure) line to apply negative pressure to a selected area to be treated. The tip cover is optionally transparent to allow the user to visually monitor the placement of the tip in a wrinkle or fissure. Water or an aqueous solution can be applied to the skin to be treated. The layer of material between the surface where microwaves are emitted and the tissue can affect the ability or efficiency of the microwaves to penetrate the heated tissue, as known in the art.

For treatment around the mouth or at the upper lip, the duration of the treatment varies by condition of the skin, but can be between about 1 minute and 20 minutes, typically with pauses to avoid excessive tissue heating. Typically, visual changes in the exterior appearance of the skin dictate the amount of treatment for a particular subject or condition.

An exemplary treatment regimen includes settings to deliver approximately 0.5-20 J/sec, using approximately 10 msec pulses with 10 msec intervals in between pulses. The treatment duration can vary from about 20 sec to about 90 sec per treatment site. This regimen is particularly suited for facial rhytides, perioral rhytides, and lentigo, especially on arms, hands, and legs. The treatment area can be cleaned and a hydrating gel and/or analgesic gel can be applied prior to treatment. During treatment the probe is gently applied against the surface of the skin to ensure electrical coupling and delivery of energy below the surface. Heating of the tissue by resistive (or ohmic) heating is generally desired in a small area of tissue, which is typically the tissue below the surface in direct contact with the distal end of the treatment tip. Methods that avoid burning or implication of physical marks at the surface of the skin are desired, and treatment regimens and varying energy pulses, pulse lengths, frequency, or all of these can accomplish this.

In another example of a device of the invention, two or more tips or delivery tips are positioned to be adjacent and to engage adjacent areas of skin. Energy setting and control is substantially the same for each tip. For a double tip aspect, two parallel and adjacent waveguides from the proximal to the distal end are fed by a single switch within the housing. This may be a single pole double throw RF switch having a single input from a coaxial waveguide with parallel outputs feeding to individual coaxial waveguides coupled to each tip.

One skilled in the art can devise and create numerous other examples according to this invention. Examples may also incorporate additional imaging, thermometry, and other elements known in the art. As but one example, the device and method disclosed herein may be employed in combination with other tissue treatment devices and methods, collectively referred to herein as "secondary tissue treatment devices," such as the use of laser, IPL, or radiofrequency devices. One skilled in the art is familiar with techniques and devices for incorporating the invention into a variety of devices and of designing improved devices though the use of the concepts presented here.

## Claims

1. A device for treating uterine tissue comprising:

   a housing having a connection point for a treatment tip and a passage for a waveguide connecting to the connection point, where the waveguide exits the housing at a grip end;
   a microwave emitting treatment tip having a distal end capable of contacting the surfaces of uterine tissue, the treatment tip functionally connected to the connection point to transfer energy from the waveguide to the distal end of the tip contacting tissue; and
   a control system for varying the pulse length, frequency, and amplitude and sending the energy to the treatment tip, the energy in a microwave frequency range of about 300 MHz to about 30 GHz and the pulses designed to create a heating effect in the tissue below the surface of the tissue.

2. The device of claim 1, wherein the microwave emitting treatment tip is replaceable.

3. The device of any one of the preceding claims, wherein bipolar microwave power is used.

4. The device of any one of the preceding claims, wherein the control system measures the energy reflection during treatment of the uterine tissue in order to adjust one or more of the amplitude, pulse number, pulse duration, or frequency of the energy delivered to the treatment tip.

5. The device of any one of the preceding claims, wherein the connection point or the tip contains a dielectric composition.

6. The device of claim 5, wherein the dielectric composition comprises Teflon.

7. The device of claim 5, wherein the impedance of the dielectric composition is selected to deliver energy to the tissue in the frequency range of about 433 MHz to about 5800 MHz.

8. The device of any one of the preceding claims, wherein the treatment tip has a convex distal tip or a pointed distal tip to contact surface of the tissue.

9. The device of any one of the preceding claims, wherein a shielding region surrounds a substantial part of the treatment tip.

**10.** The device of any one of the preceding claims, wherein the distal end of the treatment tip extends between about 1 mm and about 5 mm beyond the shielding region, preferably about 1 mm beyond the shielding region.

**11.** The device of any one of the preceding claims, wherein the treatment tip is configured to generate a microwave field within the tissue having a volume of about 10 3 mm$^3$.

**12.** The device of any one of the preceding claims for treating endometriosis, incontinence, urinary incontinence, and disorders related to these.

FIG. 1

22

10

20

FIG. 2

7

4

6

5

1

3

2

22

20

EP 2 229 907 A1

10

FIG. 3

FIG. 4

D

4    12    11    14

7    17    5    6    1

13    11    14

EP 2 229 907 A1

FIG. 5

D4

D3

5

L1

Stage 1

6

L2

Stage 2

D1

D2

EP 2 229 907 A1

FIG. 6

FIG. 7

FIG. 8

FIG. 9

10

1

22

22

4

10

22

10

22

10

22

13

EP 2 229 907 A1

FIG. 10

7

10

20

FIG. 11

10

2

20

FIG. 12

10

7

22

20

FIG. 13

EP 2 229 907 A1

FIG. 14

Europäisches
Patentamt

European
Patent Office

Office européen
des brevets

**EUROPEAN SEARCH REPORT**

Application Number

EP 10 16 6292

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 95/04385 A1 (CHEMRING LTD [GB]; FELDBERG IAN [GB]; CRONIN NIGEL [GB]; EVANS MARTYN) 9 February 1995 (1995-02-09) | 1-6,9-12 | INV. A61B18/18 |
| Y | * page 22 - page 23; figure 7 * | 7,8 | |
| Y | FR 2 699 069 A1 (SADIS BRUKER SPECTROSPIN [FR]; BRUCKER SPECTROSPIN) 17 June 1994 (1994-06-17) * page 2, line 17 - line 18 * | 7 | |
| A | FR 2 679 456 A1 (TECHNOMED INT SA [FR]) 29 January 1993 (1993-01-29) * page 3, line 3 - line 4 * | 7 | |
| A | WO 95/05869 A1 (GRUE KAARE [NO]; SHETELIG KAARE [NO]; JOHNSEN JAN FINN [NO]) 2 March 1995 (1995-03-02) * claim 5 * | 7 | |
| Y | US 2003/109862 A1 (PRAKASH MANI [US] ET AL) 12 June 2003 (2003-06-12) * paragraph [0007] - paragraph [0009]; figure 1 * | 8 | TECHNICAL FIELDS SEARCHED (IPC) A61B A61N |

The present search report has been drawn up for all claims

| Place of search | Date of completion of the search | Examiner |
|---|---|---|
| The Hague | 13 July 2010 | Petter, Erwin |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**EP 2 229 907 A1**

**ANNEX TO THE EUROPEAN SEARCH REPORT
ON EUROPEAN PATENT APPLICATION NO.**

EP 10 16 6292

This annex lists the patent family members relating to the patent documents cited in the above-mentioned European search report.
The members are as contained in the European Patent Office EDP file on
The European Patent Office is in no way liable for these particulars which are merely given for the purpose of information.

13-07-2010

| Patent document cited in search report | | Publication date | Patent family member(s) | | | Publication date |
|---|---|---|---|---|---|---|
| WO 9504385 | A1 | 09-02-1995 | AT | 161660 | T | 15-01-1998 |
| | | | AU | 684803 | B2 | 08-01-1998 |
| | | | AU | 7192794 | A | 28-02-1995 |
| | | | CA | 2163565 | A1 | 09-02-1995 |
| | | | DE | 69407598 | D1 | 05-02-1998 |
| | | | DE | 69407598 | T2 | 16-04-1998 |
| | | | EP | 0711462 | A1 | 15-05-1996 |
| | | | ES | 2113114 | T3 | 16-04-1998 |
| | | | GB | 2295094 | A | 22-05-1996 |
| | | | IN | 181894 | A1 | 24-10-1998 |
| | | | JP | 2896232 | B2 | 31-05-1999 |
| | | | JP | 9500804 | T | 28-01-1997 |
| | | | US | 6026331 | A | 15-02-2000 |
| | | | ZA | 9405453 | A | 27-03-1995 |
| FR 2699069 | A1 | 17-06-1994 | NONE | | | |
| FR 2679456 | A1 | 29-01-1993 | NONE | | | |
| WO 9505869 | A1 | 02-03-1995 | AU | 7547894 | A | 21-03-1995 |
| US 2003109862 | A1 | 12-06-2003 | EP | 1450710 | A2 | 01-09-2004 |
| | | | JP | 4437039 | B2 | 24-03-2010 |
| | | | JP | 2005507736 | T | 24-03-2005 |
| | | | WO | 03039385 | A2 | 15-05-2003 |
| | | | US | 2006282069 | A1 | 14-12-2006 |

EPO FORM P0459

For more details about this annex : see Official Journal of the European Patent Office, No. 12/82

18

**REFERENCES CITED IN THE DESCRIPTION**

**Patent documents cited in the description**

- US 11882453 B
- US 60842943 B